# EUROPÄISCHE PATENTSCHRIFT

(11) **EP 2 169 392 B1**
(45) Veröffentlichungstag und Bekanntmachung des Hinweises auf die Patenterteilung: **25.07.2018**
(21) Anmeldenummer: 08405239.8
(22) Anmeldetag: 26.09.2008
(51) Int. Cl.: G01N 27/08, G01N 33/18

(54) **Verfahren und Vorrichtung zum Messen der Härte von Wasser**
Method and device for measuring the hardness of water
Procédé et dispositif destinés à la mesure de la dureté de l'eau

(43) Veröffentlichungstag der Anmeldung: 31.03.2010
(73) Patentinhaber: R. Nussbaum AG, 4601 Olten (CH)
(72) Erfinder: Zumbrunn, Werner, 4132 Muttenz (CH); Bobst, Urs, 4703 Kestenholz (CH); Zeiter, Patrik, 4853 Riken (CH); Schreiber, Lukas, 4600 Olten (CH)
(74) Vertreter: Keller & Partner Patentanwälte AG

(56) Entgegenhaltungen:
- EP-A- 0 274 396
- DE-A1- 4 114 380
- DE-A1- 19 537 059
- DE-A1- 19 826 659
- FR-A- 2 834 509
- US-A- 4 801 551
- US-A1- 2007 024 287
- US-A1- 2007 215 531
- US-B1- 7 250 775
- MARTINHO E ET AL: "An experimental study of organic pollutant effects on time domain induced polarization measurements" JOURNAL OF APPLIED GEOPHYSICS, ELSEVIER, AMSTERDAM, NL, Bd. 60, Nr. 1, 1. September 2006 (2006-09-01), Seiten 27-40, XP025142654 ISSN: 0926-9851 [gefunden am 2006-09-01]

## Beschreibung

### Technisches Gebiet

Die Erfindung betrifft ein Verfahren zur Messung der Härte von Wasser sowie eine Vorrichtung zur Durchführung dieses Verfahrens.

### Stand der Technik

Es gibt viele Situationen, in denen die Kenntnis der Härte des Wassers hilfreich oder sogar notwendig ist. So zum Beispiel beim Einsatz von Reinigungsmitteln, deren benötigte Menge unter anderem auch von der Härte des Wassers abhängig ist. In diesem Zusammenhang werden seit einiger Zeit - besonders in Gebieten mit hartem Trinkwasser - vermehrt Entkalkungsanlagen eingesetzt.

Als Wasserhärte wird im Allgemeinen die Äquivalentkonzentration der im Wasser gelösten Ionen der Erdalkalimetalle, vor allem Kalzium und Magnesium, bezeichnet. Die Bestimmung der Wasserhärte läuft somit auf eine Bestimmung der Äquivalentkonzentration dieser Ionen hinaus. Naturgemäss, wie in der Chemie üblich, erfolgt die Bestimmung im Labor am einfachsten und genausten mit einer Titrationsmethode. Tatsächlich schreiben die meisten offiziellen Methoden zur Bestimmung der Wasserhärte wie EPA 130.2 (US Environmental Protection Agency), ASTM D 511-93 (American Society for Testing and Materials) oder ISO 6059 eine Titrationsmethode vor. Meist wird als Titrant Ethylendiamintetraacetat (EDTA) verwendet.

Viele käufliche Wasserhärteanalysatoren bilden einen Laborprozess mit einem Gerät nach; es automatisiert den ganzen Ablauf - von der Probennahme bis zur Titration und fotometrischen Bestimmung des Farbumschlagpunktes. Ein Beispiel ist das Gerät "Stamolys CA 71HA" der Firma Endress + Hauser AG, CH-4153 Reinach, oder das Gerät "Testomat 2000" der Firma CHRIST AQUA ecolife AG, CH-4147 Aesch. Diesen Geräten ist eine grosse Messgenauigkeit eigen, aber sie sind aufwendig und teuer. Überdies benötigen sie eine regelmässige Wartung - nicht zuletzt wegen der Verwendung eines Titranten.

Aus der Patentliteratur sind neben der Titrationsmethode viele andere Messverfahren für die Bestimmung der Wasserhärte bekannt. Grob lassen sie sich in die folgenden Kategorien einteilen:
- direkte oder indirekte Messung der Kalzium- oder Magnesiumionenaktivität mittels ionenselektiven Elektroden (ISE), sowie
- Verfahren, die davon Gebrauch machen, dass Austauschharze entsprechend dem Fortschritt des Ionenaustauschs ihr Volumen, ihre Farbe oder ihre Dielektrizitätskonstante ändern.
- Messung der elektrischen Leitfähigkeit als Mass für die Wasserhärte.

Als Beispiel einer Messung der Härte von Wasser mittels ionenselektiver Sensorik sei die Druckschrift DE 198 54 651 (OFS Online Fluid Sensoric) erwähnt. Diese beschreibt ein Verfahren zum Betreiben einer Wasserenthärtungsanlage mit einer Regenerationseinheit durch eine in situ-Messung der Wasserresthärte mittels eines ionenselektiven Sensors. Einem Prozesswasser wird ein Teilstrom entnommen, der via eine Ventileinheit auf eine Sensoreinheit mit mindestens einem ionenselektiven Ca-/Mg-Sensor gelenkt wird. lonenselektive Sensoren müssen regelmässig kalibriert werden. Dazu kann über die Ventileinheit aus einem Kalibrierspeicher genau definierte Kalibrierflüssigkeit auf den Sensor gegeben werden. Des Weiteren umfasst die Sensoreinheit einen TemperaturSensor. Zusammen mit der Messung der Temperatur wird mit der Hilfe der ionenselektiven Sensoren die Ca- sowie die Mg-Ionenkonzentration bestimmt. Bei zu hoher Konzentration wird ein Aktivierungssignal an die Regenerationseinheit der Wasserenthärtungsanlage gegeben. Ein Nachteil dieser Methode ist, dass die ionenselektiven Sensoren immer wieder mit einer Kalibrierflüssigkeit geeicht werden müssen. Dadurch erfordert eine Anlage, die nach einem solchen Verfahren funktioniert, einen technischen Mehraufwand bei der Konstruktion. Zudem sind solche Anlagen wartungsintensiv. Des Weiteren sind Methoden, die wie beschrieben ionenselektive Elektroden verwenden, wenig robust, weil es sich beim zentralen Prozess um einen Oberflächeneffekt handelt. Querempfindlichkeiten, Verschmutzung, Elektrodengifte etc. behindern den Einsatz dieser Verfahren in wartungsfreien Systemen.

Stellvertretend für Methoden, die Austauschharze verwenden sei die Patentschrift DE 3406724 (Spiegl) erwähnt. Diese beschreibt einen Härtefühler mit einer regenerierbaren Füllung aus ionentauschendem Harz. Dabei wird ausgenutzt, dass die Füllung bei hartem Wasser zusammenschrumpft und sich beim Regenerieren wieder ausdehnt. Die Füllung befindet sich in einem zylindrischen Behälter, wobei genügend Raum für die Volumenänderung vorgesehen ist. Die Volumenänderung wird dann mit der Hilfe eines Kolbens, der durch eine Membrane getrennt auf der Füllung sitzt, gemessen. Dieser Kolben kann einen Schalter betätigen, der zum Beispiel die Regenerierungsphase des lonentauschers auslöst. Methoden, die wie beschrieben Austauschharze verwenden, sind robuster, aber sie erlauben die Messung der absoluten Wasserhärte nicht. Sie können im Wesentlichen nur detektieren, ob ein vorgelagerter lonentauscher erschöpft ist oder nicht. Austauschharze müssen wiederum regelmässig ausgewechselt bzw. regeneriert werden.

Damit verbleiben nur Verfahren, welche auf der Messung der elektrischen Leitfähigkeit des Wassers beruhen. Stellvertretend für diese Gattung sei das Dokument DE 195 37 059 A1 (Technische Universität Dresden) erwähnt. Darin wird eine Anordnung zum berührungslosen Messen der spezifischen Leitfähigkeit wässriger Lösungen offenbart. Die Anordnung besteht aus zwei Koppelelektroden, die kapazitiv einen Wechselstrom in eine Messzelle einkoppeln. Im elektrischen Ersatzschema der Anordnung wird die Messzelle durch eine parallel geschaltete Kapazität und einen Widerstand ersetzt. In Serie geschaltet befinden sich davor und danach die beiden Kapazitäten der Koppelektroden. Ein zur ganzen Anordnung in Serie geschalteter Referenzwiderstand dient zum Messen des Stromflusses durch die Messzelle. Dabei ist der Stromfluss proportional zur Konzentration der härtebildenden Ionen in der wässrigen Lösung der Messzelle. Da die elektrische Leitfähigkeit stark von der Temperatur der wässrigen Lösung abhängt, wird der ermittelte Wert der elektrischen Leitfähig noch mit einer Temperaturfunktion korrigiert, damit auf die Härte der wässrigen Lösung geschlossen werden kann. Ein Nachteil dieser Methode ist, dass die Messung eine Summenmessung über alle in der wässrigen Lösung vorkommenden Ionenarten ist. Es gibt keine selektive Messung nur der zur Härte des Wassers beitragenden Calcium- und Magnesium-Ionen.

Vorrichtungen für die elektrischen Leitfähigkeitsmessungen können ziemlich robust ausgelegt werden, weil es sich beim Phänomen der elektrischen Leitfähigkeit um einen Volumeneffekt handelt. Allerdings sind diese Methoden in der Regel unspezifisch, weil zur elektrischen Leitfähigkeit unter anderem sowohl alle Kationen als auch alle Anionen in der Wasserprobe beitragen. Deshalb ist im Allgemeinen der Zusammenhang zwischen der elektrischen Leitfähigkeit und der Härte des Wassers schwach. Leitfähigkeitsmessungen, respektive Differenzmessungen der elektrischen Leitfähigkeit werden deshalb vornehmlich eingesetzt, um die Erschöpfung eines lonentauschers zu detektieren: Wenn er erschöpft ist, dann werden die Kalzium- und Magnesiumionen nicht mehr gegen Natriumionen ausgetauscht, was eine Änderung der Leitfähigkeit des Wassers nach sich zieht.

Eine Methode, die die Messung der elektrischen Leitfähigkeit im Hinblick auf die Bestimmung der Härte des Wassers spezifischer macht, wird in der Druckschrift DE 198 26 659 A1 (Wittmann) offenbart. Darin wird ein Prüfverfahren offenbart, mit dem man die Wirksamkeit von "physikalischen" Wasseraufbereitungsanlagen prüfen kann. Dabei wird von der Tatsache Gebrauch gemacht, dass sich der elektrische Leitwert des Wassers während des Erhitzens des Wassers ändert. Dieser Leitwert lässt sich als Funktion der Zeit darstellen. Wenn die Zeitfunktion von physikalisch aufbereitetem Wasser von derjenigen des unbehandelten Wassers abweicht, ist eine Beeinflussung des Wassers durch die physikalische Wasseraufbereitungsanlage nachgewiesen.

Das in DE 198 26 659 A1 (Wittmann) offenbarte Verfahren zeichnet sich im Wesentlichen durch drei Dinge aus:
i. Es wird nur eine behandelte mit einer unbehandelten Probe verglichen.
ii. Der Leitwert des Wassers muss während des Erhitzens dauernd gemessen werden, damit man den zeitlichen Verlauf des Leitwerts aufzeichnen und später mit dem anderen Zeitverlauf des Leitwerts vergleichen kann.
iii. Leitwertänderungen werden durch Erhitzen des Wassers verursacht.

Das gleichzeitige Behandeln der Wasserprobe, zum Beispiel durch Erhitzen, und das kontinuierliche Messen der elektrischen Leitfähigkeit respektive der Veränderung der elektrischen Leitfähigkeit auf Grund der Behandlung, ermöglicht nicht eine kontinuierliche Bestimmung der Härte des Wassers. Erst wenn die Messung der zeitlichen Änderung der elektrischen Leitfähigkeit als Folge des Erhitzens abgeschlossen ist, können die Daten verglichen und die Härte des Wassers bestimmt werden.

Aufgrund dessen lässt sich das beschriebene Verfahren auch nicht ohne weiteres in Verschnittwasseranlagen einsetzen. Solche Anlagen werden immer häufiger in Wasserleitungssystemen für den privaten Gebrauch aber auch in der Industrie eingesetzt. Sie mischen unbehandeltes Wasser derart mit enthärtetem Wasser, dass eine optimale Wasserhärte resultiert. Entsprechende Anlagen gewinnen immer mehr an Bedeutung, einerseits weil sich die Härte des unbehandelten Wassers infolge einer zunehmenden Anzahl von Wasserverbünden in kurzer Zeit in weiten Grenzen ändern kann, andererseits weil durch die Verschnittwasserregelung die Enthärtungsanlagen wirtschaftlicher betrieben werden können.

### Darstellung der Erfindung

Aufgabe der Erfindung ist es, ein dem eingangs genannten technischen Gebiet zugehörendes Verfahren und eine Vorrichtung zur Messung der Härte von Wasser zu schaffen, wobei die Bestimmung der Härte des Wassers kontinuierlich und nahezu wartungsfrei erfolgen kann. Die Lösung der Aufgabe ist durch die Merkmale des Anspruchs 1 definiert. Gemäss der Erfindung werden beim Verfahren zur Bestimmung der Härte von Wasser folgende Schritte ausgeführt:
a) Bestimmen mindestens zweier Werte der elektrischen Leitfähigkeit der Wasserprobe unter verschiedenen Bedingungen und
b) Bestimmen der Härte des Wassers der Wasserprobe aus den mindestens zwei Werten der elektrischen Leitfähigkeit.

Zur elektrischen Leitfähigkeit tragen alle im Wasser gelösten Anteile bei, das heisst sowohl Anionen als auch Kationen. Indem zwei Werte der elektrischen Leitfähigkeit unter verschiedenen Bedingungen gemessen werden, ist es möglich, jene Anteile des Wassers, das heisst insbesondere die Konzentrationen der Magnesium- und der Kalium-Ionen, genauer zu bestimmen, welche vorwiegend für die Härte des Wassers bestimmend sind. Verschiedene Bedingungen zur Messung der elektrischen Leitfähigkeit können zum Beispiel dadurch gegeben sein, dass die Wasserprobe unterschiedlich behandelt, zum Beispiel erhitzt wird, wodurch sich die elektrische Leitfähigkeit des Wassers ändert. Es ist zusätzlich möglich, dass die elektrische Leitfähigkeit unterschiedlich gemessen wird, zum Beispiel indem die (komplexe) Impedanz des Wassers bei unterschiedlichen Frequenzen bestimmt wird. Ein weiterer Vorteil ist, dass eine kontinuierliche Messung der elektrischen Leitfähigkeit und daher eine kontinuierliche Bestimmung der Härte des Wassers möglich ist. Dadurch erhält man den Vorteil, dass bei einem Verschneiden von entkalktem Wasser und kalkhaltigem Wasser Schwankungen im Härtegrad des Wassers in situ gemessen werden können und das Verschneiden dementsprechend angepasst werden kann. Das erfindungsgemässe Verfahren ist somit insbesondere für die Verwendung mit Verschnittwasseranlagen geeignet. Indem die elektrische Leitfähigkeit gemessen wird, kann weitestgehend auf Chemikalien verzichtet werden. Daher erübrigt sich auch das periodische Nachfüllen von Chemikalien in Anlagen, die nach dem erfindungsgemässen Verfahren arbeiten. Dies macht eine Anlage nahezu wartungsfrei.

Eine Vorrichtung zur Bestimmung der Härte eines Wasserstroms nach dem erfindungsgemässen Verfahren besteht aus folgenden Teilen:
a) einer Messvorrichtung zum Messen der elektrischen Leitfähigkeit des Wasserstroms,
b) einer Auswertungseinheit zum Auswerten von mindestens zwei, unter verschiedenen Bedingungen vorgenommenen Messungen der elektrischen Leitfähigkeit zum Bestimmen der Härte des Wassers.

Dabei ist es möglich, dass mit derselben Messvorrichtung zwei verschiedene Werte der elektrischen Leitfähigkeit gemessen werden, indem die Messvorrichtung die einzelnen Ionen-Anteile im Wasser, die zur elektrischen Leitfähigkeit beitragen (z. B. beteiligte Ionensorten), unterschiedlich berücksichtigt. Es ist zum Beispiel möglich, dass die Messvorrichtung die (komplexe) Impedanz des Wassers für unterschiedliche Frequenzen misst. Alternativ können mehrere Messvorrichtungen vorgesehen sein, die jeweils einen der Werte liefern.

Erfindungsgemäss gehen den oben beschriebenen Verfahrensschritten folgende Schritte voraus:
a) Aufteilen der Wasserprobe in mindestens zwei Fraktionen,
b) Unterziehen mindestens einer der Fraktionen einer Behandlung, welche die elektrische Leitfähigkeit des Wassers beeinflussen kann.

Die Bestimmung der Werte der elektrischen Leitfähigkeit des Wassers wird dann an mindestens zwei der Fraktionen vorgenommen.

Die Behandlung ist so gewählt, dass sie die einzelnen Anteile des Wassers, die zur elektrischen Leitfähigkeit beitragen (z. B. beteiligte Ionensorten), unterschiedlich beeinflusst. Somit ergeben sich durch die Behandlung unterschiedliche Werte für die elektrische Leitfähigkeit der einzelnen Fraktionen, die Aufschluss über die verschiedenen härterelevanten Anteile des Wassers geben. Dadurch sind bessere Rückschlüsse möglich, wie sich das Wasser zusammensetzt und somit kann eine genauere Angabe zur Härte des Wassers gemacht werden. In je mehr Fraktionen eine Wasserprobe aufgeteilt und unterschiedlichen Behandlungen unterworfen wird, desto genauer lässt sich im Prinzip die Härte des Wassers bestimmen.

Erfindungsgemäss wird dazu die Vorrichtung zur Bestimmung der Härte des Wassers durch folgende Einrichtungen ergänzt:
a) Eine Verteilstation zum Trennen des Wasserstroms in mindestens zwei Teilströme,
b) mindestens eine Behandlungsvorrichtung zur Beeinflussung der elektrischen Leitfähigkeit des Wassers eines Teilstroms, insbesondere ein kontinuierlicher Reaktor (Konti-Reaktor),
c) eine zweite Verteilstation, um die Fraktionen abwechslungsweise auf eine Leitfähigkeitsmesseinrichtung zu lenken, und
d) eine Messvorrichtung zum Messen der Leitfähigkeit der Teilströme.
Die Verteilstation ermöglicht das Fraktionieren der Wasserprobe. Die einzelnen Teilströme werden dann in den jeweiligen Behandlungsvorrichtungen behandelt, wodurch die elektrische Leitfähigkeit der Teilströme unterschiedlich verändert wird. Die Behandlungsvorrichtungen können aus kontinuierlichen Reaktoren (Konti-Reaktoren) bestehen, in denen die Teilströme der jeweils entsprechenden Behandlung unterworfen werden. Daraus ergibt sich der Vorteil, dass durch die verschiedenen Messresultate der elektrischen Leitfähigkeit ein genauerer Rückschluss auf die Zusammensetzung der einzelnen im Wasser gelösten Ionen-Anteile möglich ist. Dies wiederum ermöglicht eine präzisere Bestimmung der Härte des Wassers. Zudem kann die Behandlungsvorrichtung zeitlich aber auch örtlich von der Messvorrichtung getrennt sein. Dies hat wiederum den Vorteil, dass in situ gemessen werden kann. Das heisst, die einzelnen Teilströme fliessen je durch eine Behandlungsvorrichtung und danach durch die Messvorrichtung. Dabei wird die elektrische Leitfähigkeit der einzelnen Teilströme gemessen. Zur gleichen Zeit fliesst weiteres Wasser der Teilströme durch die Behandlungsvorrichtungen.

Es ist ebenfalls möglich, nicht alle Teilströme durch eine Behandlungsvorrichtung hindurchzuführen.

Mit Vorteil bleibt eine der mindestens zwei Fraktionen der Wasserprobe unbehandelt. Dadurch wird eine geringere Anzahl Behandlungen notwendig, was zu einer Vereinfachung des Verfahrens führt.

Alternativ können alle Fraktionen der Wasserprobe einer Behandlung unterzogen werden.

Mit Vorteil erfolgt die Behandlung mindestens einer Fraktion physikalisch, insbesondere durch Erhitzen, oder chemisch. Insbesondere ist auch eine elektro-chemische oder katalytische Behandlung möglich, wie sie in DE 10 2007 002 016 A1 (Sasserath) respektive EP 0 957 066 A1 (Maitron) beschrieben werden. Unterschiedliche Verfahren verändern dabei die verschiedenen Anteile des Wassers, die zur elektrischen Leitfähigkeit des Wassers beitragen. Dies wiederum lässt Rückschlüsse auf die Härte des Wassers zu. Dabei ist es möglich, dass einzelne Fraktionen auf unterschiedliche Temperaturen erhitzt werden oder dass physikalische sowie chemische, insbesondere elektro-chemische Behandlungen miteinander kombiniert werden. So kann zum Beispiel ein Teil der Fraktionen auf unterschiedliche Temperaturen erhitzt werden und der andere Teil kann einer elektro-chemischen Behandlung unterworfen werden. Auch können nur physikalische oder nur chemische Behandlungen angewendet werden.

Ablagerungen, die zum Beispiel durch Verunreinigungen im Wasser oder auf Grund der Behandlung der einzelnen Fraktionen entstehen, können die Messung der elektrischen Leitfähigkeit beeinträchtigen. Mit Vorteil werden deshalb Ablagerungen, insbesondere solche, die auf Grund der Behandlungen der einzelnen Fraktionen entstehen, mittels entsprechender und an sich bekannter Methoden, z. B. mittels Ultraschall, periodisch und automatisch entfernt. Dadurch wird die Genauigkeit der Messungen der elektrischen Leitfähigkeit bewahrt.

Ebenfalls von Vorteil ist es, wenn die Entfernung der Ablagerungen permanent erfolgt, z. B. durch die permanente Erzeugung von Ultraschallschwingungen. Inwieweit Ablagerungen periodisch oder permanent entfernt werden, wird in Abhängigkeit davon bestimmt, mit welchen Ablagerungen zu rechnen ist und welche Genauigkeit für die Messung der elektrischen Leitfähigkeit gefordert ist.

Mit Vorteil sind somit an den Teilströmen Reinigungseinheiten angebracht, mit deren Hilfe Ablagerungen entfernt werden können. Insbesondere können solche Reinigungseinheiten direkt an den Behandlungsvorrichtungen angebracht sein.

Mit Vorteil handelt es sich bei diesen Reinigungseinheiten um Ultraschallgeräte, mit deren Hilfe Ablagerungen periodisch und/oder permanent entfernt werden können. Ein Ultraschallgerät ist ein effektives und einfaches Gerät zum Entfernen von Ablagerungen, das die nachfolgende Messvorrichtung nicht beeinträchtigt.

Alternativ sind auch andere Reinigungseinheiten denkbar, die Ablagerungen zum Beispiel mechanisch, elektrochemisch oder durch die Einwirkung elektro-magnetischer Strahlung entfernen.

Alternativ, kann auch auf eine Entfernung der Ablagerungen und auf entsprechende Reinigungseinheiten verzichtet werden. Zudem können zum Beispiel Filter oder vorgeordnete Reinigungseinheiten vorgängig Verunreinigungen in der Wasserprobe entfernen, so dass keine Ablagerungen entstehen.

Wie erwähnt ist das erfindungsgemässe Verfahren besonders zum Einsatz mit Verschnittwasseranlagen geeignet. Das heisst, vorgängig wird ein Verschneiden von entkalktem Wasser mit kalkhaltigem Wasser durchgeführt. Mit Hilfe des Verfahrens kann also die Härte des Wassers nach dem Verschneiden bestimmt werden. Allfällige Schwankungen der Härte des Wassers werden dadurch sofort erkannt und das Mischverhältnis von entkalktem und kalkhaltigem Wasser kann sofort korrigiert werden. Indem die Härte des Wassers nach dem Verschneiden bestimmt wird, kann ebenfalls erkannt werden, wenn eine vorgeordnete Entkalkungsanlage, die das Wasser zum Verschneiden entkalkt, erschöpft ist oder nicht mehr ordnungsgemäss arbeitet.

Alternativ kann zuerst die Härte des Wassers bestimmt werden und danach erst das Verschneiden des Wassers erfolgen. Das Mischverhältnis wird auf Grund der Härte des kalkhaltigen Wassers gewählt. Des Weiteren kann das Verfahren zum Bestimmen der Härte des Wassers auch bei anderen Anwendungen zum Einsatz kommen. So zum Beispiel bei Waschmaschinen, die auf Grund der bestimmten Wasserhärte die Menge eines Reinigungsmittels festlegen.

Bei bevorzugten Ausführungsformen der Erfindung erfolgt die Messung der elektrischen Leitfähigkeit mittels Anlegen eines Wechselsignals, insbesondere eines Wechselstroms, bei verschiedenen Frequenzen. Da die einzelnen im Wasser gelösten Ionen-Anteile bei verschiedenen Frequenzen unterschiedlich zur elektrischen Leitfähigkeit beitragen, ist es möglich, genauer auf die einzelnen lonenkonzentrationen des Wassers zu schliessen und somit die Härte des Wassers zu bestimmen. Die Verwendung unterschiedlicher Frequenzen ist somit eine einfache und schnelle Methode. die elektrische Leitfähigkeit zu messen. Des Weiteren bietet das Anlegen eines Wechselsignals den Vorteil, dass durch das Messen der der elektrischen Leitfähigkeit bei verschiedenen Frequenzen verschiedene aussagekräftige Werte gewonnen werden können.

Zur Durchführung dieses Verfahrens besteht die Messvorrichtung aus einer Einheit, die ein Wechselsignal, insbesondere einen Wechselstrom, bei verschiedenen Frequenzen erzeugt, wodurch sich die elektrische Leitfähigkeit ermitteln lässt. Es ist möglich, die Leitfähigkeit eines Teilstromes für verschiedene Frequenzen mit der gleichen Messvorrichtung zu messen.

Alternativ sind andere Messungen der elektrischen Leitfähigkeit, zum Beispiel das Anlegen einer Gleichspannung zwischen Elektroden, vorstellbar, wonach der Stromfluss durch einen Teilstrom gemessen und so auf die elektrische Leitfähigkeit geschlossen wird.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen:
- Fig. 1: eine schematische Darstellung der Teilschritte eines ersten nicht erfindungsgemässen Verfahrens;
- Fig. 2: eine schematische Darstellung der Teilschritte eines zweiten erfindungsgemässen Verfahrens;
- Fig. 3: eine schematische Darstellung der Teilschritte eines dritten nicht erfindungsgemässen Verfahrens;
- Fig. 4: eine schematische Darstellung der Teilschritte eines vierten nicht erfindungsgemässen Verfahrens; und
- Fig. 5: eine schematische Darstellung des Einsatzes einer erfindungsgemässen Vorrichtung in einer geregelten Verschnittwasseranlage.

Grundsätzlich sind in den Figuren gleiche Teile mit gleichen Bezugszeichen versehen.

### Wege zur Ausführung der Erfindung

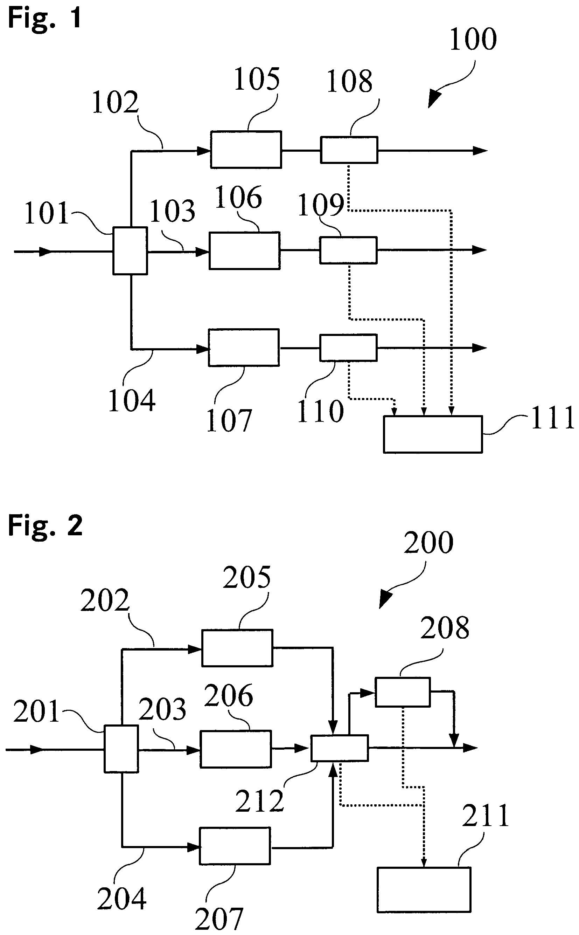

Die Figur 1 zeigt die verschiedenen Schritte einer ersten nicht erfindungsgemässen Verfahrensvariante 100. Zuerst fliesst Wasser, dessen Härte bestimmt werden soll, in eine Verteilstation 1. Diese teilt das Wasser in drei Teilströme (Fraktionen) 102, 103 und 104 auf. Jeder dieser Teilströme wird dann einer Behandlung unterzogen. Der Teilstrom 102 durchläuft einen Kühler-Erhitzer 105, der die Temperatur des Wassers auf einen Standardwert, zum Beispiel 20°C, einstellt. Der Teilstrom 103 durchläuft einen Erhitzer 106; er erwärmt das Wasser auf eine Temperatur von 70°C, bei der ein Teil des Kalziums in Form von Kalk ausgeschieden wird. Der Teilstrom 104 durchläuft einen zweiten Erhitzer 107, welcher das Wasser auf eine Temperatur von 100°C erwärmt, so dass ein grosser Teil des Kalziums in Form von Kalk ausgeschieden wird.

Die nachfolgenden Leitfähigkeitsmesseinrichtungen 108, 109 und 110 messen folglich unterschiedliche Werte für die elektrische Leitfähigkeit. Die Auswertung der drei Werte in einem Auswertegerät 111 lässt deshalb einen Rückschluss auf den ursprünglichen Gehalt der Kalziumionen im unbehandelten Wasser zu und somit kann die Härte des Wassers bestimmt werden.

Allfällige Kalkablagerungen in den Erhitzern 105, 106 und 107 lassen sich rein physikalisch entfernen, zum Beispiel durch starke akustische Einwirkung in Form von Ultraschall. Allenfalls kann die akustische Einwirkung dauerhaft und so stark gewählt werden, dass sich der Kalk gar nicht an den Wänden der Erhitzer ablagern kann, sondern im Wasser in Form von Mikropartikeln ausfällt. Diese werden vom Wasserstrom mitgetragen, tragen aber in dieser Form praktisch nichts mehr zur Leitfähigkeit bei.

Die Figur 2 zeigt eine mögliche Abfolge der verschiedenen Schritte einer ersten erfindungsgemässen Verfahrensvariante 200. Analog wie bei der ersten Verfahrensvariante 100 wird das zu messende Wasser in einer Verteilstation 202 auf verschiedene Teilströme 202, 203 und 204 aufgeteilt und auf die verschiedenen Behandlungseinrichtungen 205, 206 und 207 gelenkt. Bei dieser Verfahrensvariante 200 wird im Unterschied zur ersten Verfahrensvariante 100 aber nur eine Leitfähigkeitsmesseinrichtung 208 eingesetzt. Nach der Behandlung der Teilströme 205, 206 und 207 werden diese nämlich mittels Ventile einer zweiten Verteilstation 212 abwechslungsweise auf die Leitfähigkeitsmesseinrichtung 208 gelenkt, wo der entsprechende Leitfähigkeitswert gemessen wird. Der entsprechende Wert wird dann von der Leitfähigkeitsmesseinrichtung 208 an ein Auswertegerät 211 weitergegeben, wo dann die Härte des Wassers bestimmt wird. Der Vorteil dieser Verfahrensvariante 200 besteht darin, dass nur eine Leitfähigkeitsmesseinrichtung 208 benötigt wird. Dadurch werden Nullpunktfehler eliminiert, was insbesondere eine deutlich genauere Bestimmung von Leitfähigkeitsdifferenzen ermöglicht. Die Bestimmung der Härte des Wassers ist immer noch in situ möglich. Kurzzeitige Schwankungen der Härte des Wassers sind aber nicht mehr detektierbar. Bei zeitlich langsamen Veränderungen der Wasserhärte, wie sie bei der öffentlichen Trinkwasserversorgung auftreten, ist dies jedoch nicht problematisch.

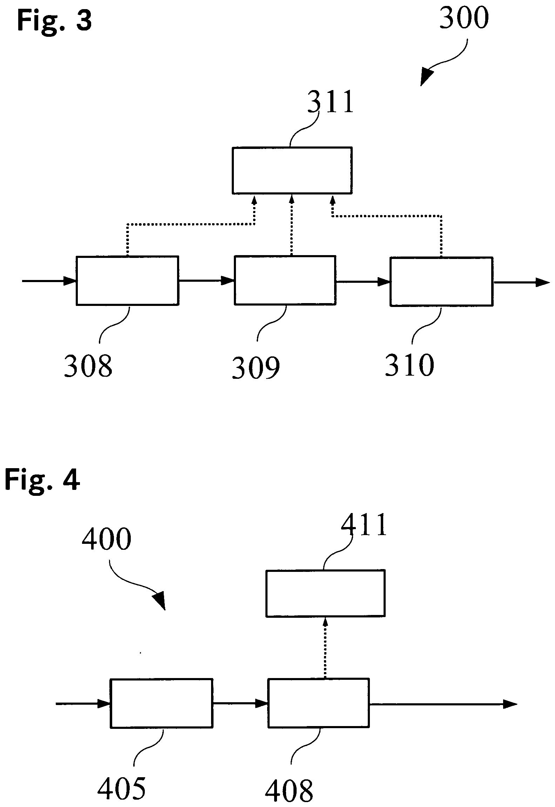

Die Figur 3 zeigt eine mögliche Abfolge der verschiedenen Schritte einer dritten nicht erfindungsgemässen Verfahrensvariante 300. Bei dieser Verfahrensvariante 300 wird das Wasser, dessen Härte bestimmt werden soll, weder aufgeteilt noch durchläuft es einen Erhitzer oder eine andere Form einer Behandlungseinheit, sondern es wird direkt durch mehrere Messvorrichtungen 308, 309, 310 geleitet, die die elektrische Leitfähigkeit des Wassers bei jeweils unterschiedlichen Frequenzen messen, d. h. es werden zur Leitfähigkeitsbestimmung Wechselspannungen bzw. -ströme mit unterschiedlicher Schwingungsfrequenz eingesetzt. Die Resultate der einzelnen Messungen werden dann in einem Auswertegerät 311 ausgewertet. Je nach Situation, das heisst falls mit Temperaturschwankungen des Wassers zu rechnen ist, kann eine Temperaturmesseinheit (nicht gezeichnet) vorhanden sein, deren Messung im Auswertegerät 311 ebenfalls berücksichtigt wird. Anstelle der Temperaturmesseinheit kann auch eine Regulierungseinheit (nicht gezeichnet) vorhanden sein, die sicherstellt, dass die Temperatur des Wassers konstant bleibt.

Die Figur 4 zeigt eine mögliche Abfolge der verschiedenen Schritte einer vierten nicht erfindungsgemässen Verfahrensvariante 400. Wie in der Verfahrensvariante 300 wird auch in der vierten Verfahrensvariante 400 das zu messende Wasser nicht aufgeteilt. Im Unterschied zur dritten Verfahrensvariante 300, durchläuft das Wasser aber eine Behandlungseinrichtung 405 und danach eine Leitfähigkeitsmesseinrichtung 408. Indem die Behandlungseinrichtung 405 periodisch ein- respektive ausgeschaltet wird, durch läuft in zeitlichen Intervallen behandeltes und unbehandeltes Wasser die Leitfähigkeitsmesseinrichtung 408. Die Leitfähigkeitsmesseinrichtung 408 gibt die Werte an ein Auswertegerät 411 weiter, das dann die Härte des Wassers bestimmt. Alternativ, anstatt nur die Behandlungseinrichtung 405 ein- und auszuschalten, kann die Behandlungseinrichtung 405 ihre Behandlung zeitlich ändern. So kann zum Beispiel ein Erhitzer das Wasser auf unterschiedliche Temperaturen erhitzen. Dabei ist darauf zu achten, dass die entsprechende Temperatur auch lange genug gehalten wird, damit der entsprechende, zur dieser Temperatur gehörende Leitfähigkeitswert des Wassers gemessen werden kann.

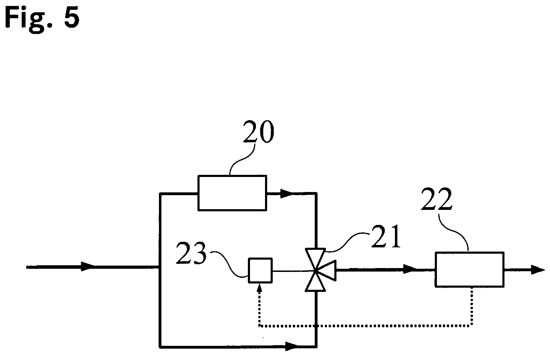

Die Figur 5 zeigt eine Verschnittwasseranlage, das heisst eine Anlage, die unbehandeltes Wasser mit weichem Wasser so vermischen kann, dass Wasser mit konstanter, optimaler Härte resultiert. Die Anlage beinhaltet neben einem Wasserenthärter 20, zum Beispiel in Form eines Kationentauschers, ein steuerbares Mischventil 21, einen Regler 23 und eine Vorrichtung 22, die gemäss dem erfindungsgemässen Verfahren, gemäss der Verfahrensvariante 200, die Härte des Wassers bestimmt. Dass die Vorrichtung 22 nach dem Mischventil angeordnet ist, hat den Vorteil, dass nur Abweichungen vom Sollwert der Wasserhärte zu detektieren sind, mit anderen Worten, dass die Vorrichtung 22 zwecks einer optimalen Regelung nicht die allenfalls in weiten Grenzen schwankende Härte des unbehandelten Wassers messen muss. Ein weiterer Vorteil ist, dass mit der Anlage auch die Erschöpfung des Kationentauschers 20 detektiert werden kann. Vorausgesetzt, dass die Härte des unbehandelten Wassers über dem Sollwert der Härte des Mischwassers liegt, kündigt sich die Erschöpfung an oder ist bereits eingetreten, wenn das Mischventil beinahe nur noch Wasser vom lonentauscher 20 zudosiert oder ausschliesslich solches zudosiert.

Im Allgemeinen können Messvorrichtungen, die einen Wechselstrom anlegen, so ausgestattet sein, dass dieselbe Messvorrichtung die elektrische Leitfähigkeit für zwei verschiedene Frequenzen misst. Dabei kann eine Messeinrichtung bei mehreren Frequenzen messen oder es ist auch möglich, dass mehrere Messvorrichtungen vorhanden sind, die mit Hilfe von Wechselstrom die elektrische Leitfähigkeit für verschiedene Frequenzen gleichzeitig messen. Als Frequenzbereiche kommt insbesondere der Bereich von 1 kHz bis 100 MHz, vorteilhaft von 10 kHz bis 200 kHz in Frage. Frequenzen darüber und darunter sind aber ebenfalls möglich.

Die Temperatur beim Erhitzen zur Behandlung der einzelnen Fraktionen kann sich grundsätzlich über den ganzen Bereich erstrecken, bei dem Wasser beim jeweils herschenden Druck flüssig ist. Damit die Wasserprobe nicht gekühlt werden muss und somit eine teure Kühlvorrichtung vorhanden sein muss, ist eine untere Temperatur von 20°C vorteilhaft.

Die einzelnen Temperaturen, auf welche die Fraktionen erhitzt werden, sollten so gewählt werden, dass ein deutlicher Unterschied in der elektrischen Leitfähigkeit nach der Behandlung messbar ist.

Zusammenfassend ist festzustellen, dass durch die Erfindung ein einfaches Verfahren sowie eine Vorrichtung geschaffen werden, mit deren Hilfe kontinuierlich die Härte von Wasser bestimmt werden kann. Dabei wird weitgehend auf den Zusatz von Chemikalien verzichtet, was zu einer wartungsarmen Vorrichtung führt.

## Patentansprüche

1. Verfahren zur Bestimmung der Härte von Wasser, wobei mit einer Wasserprobe folgende Schritte ausgeführt werden:
a) Bestimmen mindestens zweier Werte der elektrischen Leitfähigkeit der Wasserprobe unter verschiedenen Bedingungen und
b) Bestimmen der Härte des Wassers der Wasserprobe aus den mindestens zwei Werten der elektrischen Leitfähigkeit,
wobei vorgängig folgende zwei Schritte ausgeführt werden:
c) Aufteilen der Wasserprobe in mindestens zwei Fraktionen (202, 203, 204),
d) Unterziehen mindestens einer der Fraktionen einer Behandlung, welche die elektrische Leitfähigkeit des Wassers beeinflussen kann,
**dadurch gekennzeichnet, dass**
e) nach der Behandlung der Fraktionen (202, 203, 204) diese mittels Ventile einer zweiten Verteilstation (212) abwechslungsweise auf eine Leitfähigkeitsmesseinrichtung (208) gelenkt werden, wobei
f) die Bestimmung der Werte der elektrischen Leitfähigkeit an mindestens zwei der Fraktionen (202, 203, 204) vorgenommen wird.

2. Verfahren nach Anspruch 1, **dadurch gekennzeichnet, dass** mindestens eine der Fraktionen (202, 203, 204) unbehandelt bleibt.

3. Verfahren nach Anspruch 1 oder 2, **dadurch gekennzeichnet, dass** die Behandlung mindestens einer der Fraktionen (202, 203, 204) physikalisch, insbesondere durch Erhitzen, erfolgt.

4. Verfahren nach einem der Ansprüche 1 bis 3, **dadurch gekennzeichnet, dass** die Behandlung mindestens einer der Fraktionen (202, 203, 204) chemisch, insbesondere elektro-chemisch oder katalytisch, erfolgt.

5. Verfahren nach einem der Ansprüche 1 bis 4, **dadurch gekennzeichnet, dass** Ablagerungen, die infolge einer Wasserbehandlung entstehen, mittels entsprechender Methoden, insbesondere mit Ultraschall, periodisch und automatisch entfernt werden.

6. Verfahren nach einem der Ansprüche 1 bis 4, **dadurch gekennzeichnet, dass** Ablagerungen, die infolge einer Wasserbehandlung entstehen, durch permanente Anwendung entsprechender Methoden, insbesondere mit Ultraschall, verhindert werden.

7. Verfahren nach einem der Ansprüche 1 bis 6, **dadurch gekennzeichnet, dass** vorgängig ein Verschneiden von entkalktem Wasser mit kalkhaltigem Wasser durchgeführt wird.

8. Verfahren nach einem der Ansprüche 1 bis 7, **dadurch gekennzeichnet, dass** zur Bestimmung der mindestens zwei Werte der elektrischen Leitfähigkeit Messungen mithilfe von einem Wechselsignal, insbesondere einem Wechselstrom, bei verschiedenen Frequenzen vorgenommen werden.

9. Vorrichtung zur Bestimmung der Härte des Wassers eines Wasserstroms nach einem Verfahren nach einem der Ansprüche 1 bis 8, bestehend aus:
a) einer Messvorrichtung (208) zum Messen der elektrischen Leitfähigkeit des Wasserstroms,
b) einer Auswertungseinheit zum Auswerten von mindestens zwei, unter verschiedenen Bedingungen vorgenommenen Messungen der elektrischen Leitfähigkeit zum Bestimmen der Härte des Wassers,
c) eine Verteilstation zum Trennen des Wasserstroms in mindestens zwei Teilströme (202, 203, 204),
d) mindestens eine Behandlungsvorrichtung (205, 206, 207) zur Beeinflussung der elektrischen Leitfähigkeit des Wassers eines Teilstroms, insbesondere ein kontinuierlicher Reaktor (Konti-Reaktor)
e) eine Verteilstation (212) mit Ventilen zum abwechslungsweisen Lenken der Teilströme (202, 203, 204) auf die Messvorrichtung (208).

10. Vorrichtung nach Anspruch 9, **dadurch gekennzeichnet, dass** Reinigungseinheiten zum Entfernen von Ablagerungen, insbesondere von Ablagerungen in den Behandlungseinheiten, vorhanden sind.

11. Vorrichtung nach Anspruch 10, **dadurch gekennzeichnet, dass** die Reinigungseinheiten ein Ultraschallgerät beinhalten.

12. Vorrichtung nach einem der Ansprüche 9 bis 11, **dadurch gekennzeichnet, dass** die Messvorrichtung (208, 209, 210) eine Einrichtung zur Erzeugung von Wechselsignalen, insbesondere Wechselströmen, voneinander verschiedener Frequenzen umfasst und dass sie derart ausgebildet ist, dass die Leitfähigkeit bei den verschiedenen Frequenzen messbar ist.

## Claims

1. Method for determining the hardness of water, wherein the following steps are carried out with a water sample:
a) determining at least two values of the electrical conductivity of the water sample under different conditions, and
b) determining the hardness of the water of the water sample from the at least two values of the electrical conductivity,
wherein the following two steps are carried out in advance:
c) dividing the water sample into at least two fractions (202, 203, 204),
d) subjecting at least one of the fractions to a treatment which can influence the electrical conductivity of the water,
**characterized in that**
e) after the treatment of the fractions (202, 203, 204), these are alternately directed to a conductivity measurement device (208) by means of valves of a second distribution station (212), wherein
f) the values of the electrical conductivity are determined at at least two of the fractions (202, 203, 204).

2. Method according to Claim 1, **characterized in that** at least one of the fractions (202, 203, 204) remains untreated.

3. Method according to Claim 1 or 2, **characterized in that** the treatment of at least one of the fractions (202, 203, 204) is realized physically, in particular by heating.

4. Method according to one of Claims 1 to 3, **characterized in that** the treatment of at least one of the fractions (202, 203, 204) is realized chemically, in particular electrochemically or catalytically.

5. Method according to one of Claims 1 to 4, **characterized in that** deposits which form as a result of a water treatment are removed periodically and automatically by means of corresponding methods, in particular by way of ultrasound.

6. Method according to one of Claims 1 to 4, **characterized in that** deposits which form as a result of a water treatment are prevented through the permanent use of corresponding methods, in particular by way of ultrasound.

7. Method according to one of Claims 1 to 6, **characterized in that** decalcified water is mixed with hard water in advance.

8. Method according to one of Claims 1 to 7, **characterized in that**, in order to determine the at least two values of the electrical conductivity, measurements are performed with the aid of an alternating signal, in particular an alternating current, at different frequencies.

9. Device for determining the hardness of the water of a water stream according to a method according to one of Claims 1 to 8, consisting of:
a) a measurement device (208) for measuring the electrical conductivity of the water stream,
b) an evaluation unit for evaluating at least two measurements, performed under different conditions, of the electrical conductivity for determining the hardness of the water,
c) a distribution station for separating the water stream into at least two partial streams (202, 203, 204),
d) at least one treatment device (205, 206, 207) for influencing the electrical conductivity of the water of a partial stream, in particular a continuous reactor,
e) a distribution station (212) having valves for alternately directing the partial streams (202, 203, 204) to the measurement device (208).

10. Device according to Claim 9, **characterized in that** cleaning units for removing deposits, in particular deposits in the treatment units, are present.

11. Device according to Claim 10, **characterized in that** the cleaning units contain an ultrasound unit.

12. Device according to one of Claims 9 to 11, **characterized in that** the measurement device (208, 209, 210) comprises an apparatus for generating alternating signals, in particular alternating currents, of mutually different frequencies, and **in that** it is configured such that the conductivity is able to be measured at the different frequencies.

## Revendications

1. Procédé de détermination de la dureté de l'eau, dans lequel on exécute les opérations suivantes avec un échantillon d'eau:
a) déterminer au moins deux valeurs de la conductibilité électrique de l'échantillon d'eau dans des conditions différentes et
b) déterminer la dureté de l'eau de l'échantillon d'eau à partir desdites au moins deux valeurs de la conductibilité électrique,
dans lequel on exécute d'abord les deux opérations suivantes:
c) répartir l'échantillon d'eau en au moins deux fractions (202, 203, 204),
d) soumettre au moins une des fractions à un traitement, qui peut influencer la conductibilité électrique de l'eau,
**caractérisé en ce que**
e) après le traitement des fractions (202, 203, 204), on dévie celles-ci au moyen de soupapes d'une deuxième station de répartition (212) en alternance sur un dispositif de mesure de la conductibilité (208), dans lequel
f) on effectue la détermination des valeurs de la conductibilité électrique sur au moins deux des fractions (202, 203, 204).

2. Procédé selon la revendication 1, **caractérisé en ce qu'**au moins une des fractions (202, 203, 204) reste non traitée.

3. Procédé selon la revendication 1 ou 2, **caractérisé en ce que** l'on effectue le traitement d'au moins une des fractions (202, 203, 204) par voie physique, en particulier par chauffage.

4. Procédé selon l'une quelconque des revendications 1 à 3, **caractérisé en ce que** l'on effectue le traitement d'au moins une des fractions (202, 203, 204) par voie chimique, en particulier par voie électrochimique ou catalytique.

5. Procédé selon l'une quelconque des revendications 1 à 4, **caractérisé en ce que** l'on élimine périodiquement et automatiquement, par des méthodes correspondantes, en particulier par ultrasons, des dépôts qui apparaissent à la suite d'un traitement de l'eau.

6. Procédé selon l'une quelconque des revendications 1 à 4, **caractérisé en ce que** l'on empêche, par l'application permanente de méthodes correspondantes, en particulier des ultrasons, des dépôts qui apparaissent à la suite d'un traitement de l'eau.

7. Procédé selon l'une quelconque des revendications 1 à 6, **caractérisé en ce que** l'on opère préalablement un mélange d'eau décalcarisée avec de l'eau contenant du calcaire.

8. Procédé selon l'une quelconque des revendications 1 à 7, **caractérisé en ce que** pour la détermination desdites au moins deux valeurs de la conductibilité électrique, on effectue des mesures à l'aide d'un signal alternatif, en particulier un courant alternatif, à différentes fréquences.

9. Dispositif pour la détermination de la dureté de l'eau d'un échantillon d'eau par un procédé selon l'une quelconque des revendications 1 à 8, se composant de:
a) un dispositif de mesure (208) pour mesurer la conductibilité électrique du courant d'eau,
b) une unité d'évaluation pour évaluer au moins deux mesures de la conductibilité électrique, effectuées dans des conditions différentes, pour la détermination de la dureté de l'eau,
c) une station de répartition pour séparer le courant d'eau en au moins deux courants partiels (202, 203, 204),
d) au moins un dispositif de traitement (205, 206, 207) pour influencer la conductibilité électrique de l'eau d'un courant partiel, en particulier un réacteur continu (conti-réacteur),
e) une station de répartition (212) avec des soupapes pour la déviation alternée des courants partiels (202, 203, 204) sur le dispositif de mesure (208).

10. Dispositif selon la revendication 9, **caractérisé en ce qu'**il se trouve des unités de nettoyage pour éliminer des dépôts, en particulier des dépôts dans les unités de traitement.

11. Dispositif selon la revendication 10, **caractérisé en ce que** les unités de nettoyage contiennent un appareil à ultrasons.

12. Dispositif selon l'une quelconque des revendications 9 à 11, **caractérisé en ce que** le dispositif de mesure (208, 209, 210) comprend un dispositif pour produire des signaux alternatifs, en particulier des courants alternatifs, de fréquences différentes l'une de l'autre, et **en ce qu'**il est conçu de telle manière que la conductibilité puisse être mesurée aux différentes fréquences.
